# EUROPEAN PATENT SPECIFICATION

(11) **EP 3 495 820 B1**
(45) Date of publication and mention of the grant of the patent: **27.01.2021**
(21) Application number: 18211204.5
(22) Date of filing: 10.12.2018
(51) Int. Cl.: G01N 33/569

(54) **AN IMPROVED METHOD FOR DIAGNOSING A FUNGAL INFECTION**
VERBESSERTES VERFAHREN ZUR DIAGNOSE EINER PILZINFEKTION
PROCÉDÉ AMÉLIORÉ PERMETTANT DE DIAGNOSTIQUER UNE INFECTION FONGIQUE

(30) Priority: 11.12.2017 EP 17206386
(43) Date of publication of application: 12.06.2019
(73) Proprietor: Euroimmun Medizinische Labordiagnostika AG, 23560 Lübeck (DE)
(72) Inventor: Herbst, Victor, 23628 Krummesse (DE); Hoffmann, Kathrin, 23558 Lübeck (DE)

(56) References cited:
- CHRISTOPHER R THORNTON: "Development of an Immunochromatographic Lateral-Flow Device for Rapid Serodiagnosis of Invasive Aspergillosis", CLINICAL AND VACCINE IMMUNO, AMERICAN SOCIETY FOR MICROBIOLOGY, WASHINGTON, DC, US, vol. 15, no. 7, 1 July 2008 (2008-07-01), pages 1095-1105, XP007912963, ISSN: 1556-6811, DOI: 10.1128/CVI.00068-08 [retrieved on 2008-05-07]
- Grace Megan Howells: "Monoclonal Antibody ED1 as a Diagnostic Antibody for Human Invasive Pulmonary Aspergillosis", , 7 October 2017 (2017-10-07), XP055442975, Retrieved from the Internet: URL:https://ore.exeter.ac.uk/repository/ha ndle/10871/27796 [retrieved on 2018-01-22]
- Anonymous: "DOUBLE ANTIBODY SANDWICH ELISA (DAS-ELISA)", LABORATORY PROCEDURES FOR PLANT CELL VIRUSES, 1 January 1998 (1998-01-01), XP055442976, Retrieved from the Internet: URL:http://www.cabri.org/guidelines/plant_ virus/pcv204ap2.html [retrieved on 2018-01-22]
- N. P. Wiederhold ET AL: "Interlaboratory and Interstudy Reproducibility of a Novel Lateral-Flow Device and Influence of Antifungal Therapy on Detection of Invasive Pulmonary Aspergillosis", JOURNAL OF CLINICAL MICROBIOLOGY, vol. 51, no. 2, 1 February 2013 (2013-02-01), pages 459-465, XP55659870, US ISSN: 0095-1137, DOI: 10.1128/JCM.02142-12

## Description

The present invention relates to a method for diagnosing an *Aspergillus* infection in a patient, comprising the step detecting in a sample beta-1,5-galactofuranose from mycelium of a pathogenic *Aspergillus* strain, wherein the sample is contacted with a first antibody binding specifically to said antigen and wherein any antigen bound to said first antibody is detected by way of an immunoassay selected from the group consisting of an ELISA, wherein the first antibody is coated on a microplate, and a bead-based assay, wherein the first antibody is coated on a bead, and a use of an antibody binding to beta-1,5-galactofuranose from mycelium of a pathogenic *Aspergillus* strain for increasing the reliability of an immunoassay for the diagnosis of an *Aspergillus* infection in a patient wherein the sample is contacted with a first antibody to said antigen followed by detection of any antigen bound to said first antibody by way of an immunoassay selected from the group consisting of an ELISA, wherein the first antibody is coated on a microplate, and a bead-based assay, wherein the first antibody is coated on a bead, wherein the sample is a serum sample, characterized in that the sample is subjected to a denaturing step prior to contacting the sample with the antibody. The immunoassay is not a membrane-based immunoassay selected from the group consisting of lateral flow device, western blot, dot blot and line blot.

Invasive aspergillosis (IA) is an important cause of morbidity and mortality in patients with haematological malignancies and profound immuno-suppression, for example after haematopoietic stem cell transplantation, but also non-haematological patients, for example those suffering from a condition selected from the group comprising diabetes mellitus, systemic lupus erythematosus, old pulmonary tuberculosis and transplantation of liver or kidney.

Rapid detection of IA using immunodiagnostics has centred around the detection of fungal galactomannan (GM) (Latge et al. (1994) Chemical and immunological characterization of the extracellular galactomannan secreted by Aspergillus fumigatus. Infect. Immun. 62:5424-5433; Pazos, et al. (2005) Contribution of (1∼3)-ß-D-glucan chromogenic assay to diagnosis and therapeutic monitoring of invasive aspergillosis in neutropenic adult patients: a comparison with serial screening for circulating galactomannan. J. Clin. Microbiol. 43:299-305; Quindos (2006) New microbiological techniques for the diagnosis of invasive mycoses caused by filamentous fungi. Clin. Microbiol. Infect. 12:40-52). GM is a branched carbohydrate comprising a mannose main chain and short side chains comprising a galactose molecule.

Monoclonal antibodies (mAbs) have been successfully used in the detection of GM, and they form the basis of commercial laboratory based tests such as the Platelia *Aspergillus* ELISA kit that incorporates a rat mAb (EB-A2) directed against tetra (1∼5)-ß-D galactofuranoside, the immunodominant epitope in the antigen (Morelle et al. (2005) Galactomannoproteins of Aspergillus fumigatus. Euk. Cell 4: 1308-1316; Stynen et al. (1995) A new sensitive sandwich enzyme linked immunosorbent assay to detect galactofuran in patients with invasive aspergillosis. J. Clin. Microbiol. 33:497-500 ; Stynen et al. (1992) Rat monoclonal antibodies against Aspergillus galactomannan. Inf. Immun. 60:2237-2245).

Immunoassays for GM detection are a significant asset for managing patients at risk of catching IA because of detection of the antigen in the early stages of disease progression. Despite their widespread use, recent studies have revealed significant variation in performance.

False positive results have been attributed to cross-reaction of mAb EB-A2 with various reagents such as GM from *non-Aspergillus* fungi (Giacchino et al. (2006) Aspergillus galactomannan enzyme-linked immunosorbent assay cross-reactivity caused by invasive Geotrichum capitatum. J. Clin. Microbiol. 44:3432-3434; Kappe et al. (1993) New cause for false positive results with the Pastorex Aspergillus antigen latex agglutination test. J. Clin. Microbiol. 31 :2489-2490, Quindos (2006) New microbiological techniques for the diagnosis of invasive mycoses caused by filamentous fungi. Clin. Microbiol. Infect. 12:40-52; Swanink et al. (1997) Specificity of the sandwich enzyme-linked immunosorbent assay for detecting Aspergillus galactomannan. J. Clin. Microbiol. 35:257-260; Verweij et al. (2006) Issues with galactomannan testing. Med. Mycol. 44: 179 183), and galactoxylomannan from *Cryptococcus neoformans;* Dalle et al. (2005) Cryptococcus neoformans galactoxylomannan contains an epitope(s) that is cross-reactive with Aspergillus galactomannan. J. Clin. Microbiol. 43:2929-2931.).

Whilst several authors have reported satisfactory sensitivities of >85%, sensitivity of the assay can vary considerably and was reported as low as 29% (Verweij et al. (2006) Issues with galactomannan testing. Med. Mycol. 44: 179-183). Several lines of evidence suggest that the sensitivity very much depends on the cohort of patients chosen and the way they have been treated. For example, lower sensitivities have been reported with samples from patients treated with posaconazol (Marr et al. (2005). Antifungal Therapy Decreases Sensitivity of the Aspergillus Galactomannan Enzyme Immunoassay. Clin Infect Dis. 40, 1762-1769.) The reason is unknown. It seems that many authors reporting higher sensitivities used serum samples from cohorts excluding such patients.

Another immunoassay was developed by Thornton (2008) (Development of an Immunochromatographic Lateral-Flow Device for Rapid Serodiganosis of Invasive Aspergillosis, Clin. And Vacc. Immun. 15-7, 1095-1105). It is based on the use of the monoclonal antibody JF5 to the *Aspergillus* JF5 antigen in a Lateral Flow Device, which was later identified as beta-1,5-galactofuranose. However, such devices are hardly useful for the high-throughput screening of samples in routine diagnostics. Furthermore, the sensitivity of this assay, according to this study, leaves significant room for improvement, as a range of false-negative results were obtained.

The same assay has been disclosed in WO2010/082034, but both the GM ELISA and the JF5 Lateral Flow Device assay were reported to have essentially the same sensitivity (100%). Since samples from artificially infected Guinea pigs were used, these results cannot be translated to humans, and the fact remains that there remains room for improving the sensitivities of the assays for use in the diagnosis of humans. WO2010/082034 discloses an ELISA assay, but only for checking the quality of antibody using coated antigen, not a diagnostic assay based on human samples, let alone a boiling step.

Consistent with this, when Hoenigl et al. (2014) (Performance of Galactomannan, Beta-D-Glucan, Aspergillus Lateral-Flow-Device, Conventional Culture and PCR Tests with Bronchoalveolar Lavage Fluid for Diagnosis of Invasive Pulmonary Aspergillosis, J. Clin. Microbiol 52-6, 2039-2045) compared these methods using human samples, significantly lower sensitivities were reported, more specifically 80% for both assays.

Davies et al. (2017) (Towards translational ImmunoPET/MR Imaging of Invasive Pulmonary Aspergillosis: The Humanised Monoclonal Antibody JF5 Detects Aspergillus Lung Infections In Vivo, Theranostics, 7-4, 3398-3414) reported the use of humanized monoclonal antibody hJF5 for the *in vivo* detection of *Aspergillus* infections. As part of their investigations, they used monoclonal and humanized JF5 antibodies to detect *Aspergillus* infections in murine, but not human samples. Besides, murine lung tissue obtained by excision of the lungs were examined. The diagnostic reliability of the antibodies for routine high-throughput screening of human samples was not addressed. It is not disclosed that the samples were subjected to boiling.

Ku *et al.* (2012) report a rather unsatisfactory performance of the GMA ELISA with samples from non-haematological patients. The sensitivity was 23.1%, a value considered "very low" by the authors.

The abstract of the thesis "Monoclonal Antibody ED1 as a Diagnostic Antibody for Human Invasive Pulmonary Aspergillosis" by Grace Megan Howells (http://hdl.handle.net/10871/27796) discloses the characterization of a newly developed murine mAb ED1 and its use in combination with antibody JF5 in an immunoassay format to detect invasive pulmonary aspergillosis in a guinea pig model of the disease.

Therefore, a problem underlying the present invention is to provide an improved immunoassay selected from the group consisting of an ELISA and a bead-based assay, that overcomes shortcomings of conventional assays, in particular lack of reproducibility, specificity and sensitivity, and may preferably be used for high-throughput screening of samples.

Another problem underlying the present invention is to overcome shortcomings of conventional assays, in particular increasing specificity and sensitivity, with regard to patients suffering from CGD (chronic granulomatous disease) and/or hyper-lgE syndrome with recurrent infection (HIES; Job syndrome).

Another problem underlying the present invention is to provide a diagnostically reliable assay, in particular having optimized specificity and/or sensitivity, that may be used in a high-throughput format for a multitude of samples in an automated manner.

Another problem underlying the present invention is to provide a diagnostically reliable assay for high-throughput screening having an improved sensitivity when used to test samples from patients treated with anti-fungal drugs such as posaconazole or non-haematooncological patients.

Another problem underlying the present invention is to provide a diagnostically reliable assay, wherein a positive diagnosis can be accomplished at an early stage of the infection and/or a late stage of the infection, in particular earlier or later, respectively compared to state of the art assays, leading to a an extended period in which a reliable diagnosis based on a serological assay is possible.

The problem underlying the present invention is solved by the subject matter of the attached independent and dependent claims.

In a first aspect, the problem underlying the present invention is solved by a
method for diagnosing an *Aspergillus* infection in a patient, comprising the step detecting in a sample beta-1,5-galactofuranose from mycelium of a pathogenic *Aspergillus* strain,
wherein the sample is contacted with a first antibody binding specifically to said antigen and
wherein any antigen bound to said first antibody is detected by way of an immunoassay selected from the group consisting of an ELISA, wherein the first antibody is coated on a microplate, and a bead-based assay, wherein the first antibody is coated on a bead
wherein the immunoassay is not a membrane-based immunoassay selected from the group consisting of lateral flow device, western blot, dot blot and line blot,
wherein the sample is a serum sample,
characterized in that the sample is subjected to a denaturing step prior to contacting the sample with the antibody.

In a second aspect, the problem underlying the present invention is solved by a use of an antibody binding specifically to beta-1,5-galactofuranose from mycelium of a pathogenic *Aspergillus* strain for increasing the diagnostic reliability of an immunoassay for the diagnosis of an *Aspergillus* infection in a patient comprising the step detecting said antigen in a sample,
wherein the sample is contacted with a first antibody to said antigen followed by detection of any antigen bound to said first antibody by way of an immunoassay selected from the group consisting of an ELISA, wherein the first antibody is coated on a microplate, and a bead-based assay, wherein the first antibody is coated on a bead
wherein the immunoassay is not a membrane-based immunoassay selected from the group consisting of lateral flow device, western blot, dot blot and line blot,
wherein the sample is a serum sample,
characterized in that the sample is subjected to a denaturing step prior to contacting the sample with the antibody.

In a preferred embodiment the immunoassay is a sandwich ELISA.

In a preferred embodiment, the first antibody is a monoclonal antibody.

In a first embodiment the antibody is the antibody JF5 or a variant thereof.

In a preferred embodiment, the *Aspergillus* strain is selected from the group comprising *A*. *fumigatus, A. flavus, A. niger, A. terreus, A. nidulans* and is preferably *A. fumigatus AF293.*

In a preferred embodiment, the sample is a serum sample.

In a preferred embodiment, the method is for diagnosing an invasive aspergillosis.

In a preferred embodiment, the sample is subjected to a denaturing step, preferably a boiling step prior to contacting the sample with the antibody.

In a preferred embodiment, the antigen is detected using a second antibody, which is preferably the same antibody as the first antibody.

In a preferred embodiment, the use is for increasing the sensitivity.

In a preferred embodiment, the use is for increasing the diagnostic reliability, preferably sensitivity or specificity, at an early stage of the infection.

The present invention is also based on the inventors' surprising finding that denaturing a sample, for example by way of boiling, increases the diagnostic reliability of the test, in particular the specificity at low concentrations including the absence of the antigen such that false positive results may be avoided. The authors theorize that this releases the antigen which may otherwise be masked by other components or compete for binding with other components in the samples or denatures other components in the sample having unwanted cross-reactivity which may lead to false positive reactions.

In a preferred embodiment, the antibody used binds to beta-1,5-galactofuranose. This may be purified beta-1,5-galactofuranose, but it is preferably beta-1,5-galactofuranose from the mycelium of an infectious Aspergillus strain, where it may be associated with larger macromolecules such as carbohydrates carrying more than one molecule of beta-1,5-galactofuranose. Such antigenic beta-1,5-galactofuranose from mycelium may be isolated as disclosed for what is referred to as "JF5 antigen" in WO2010082034.

The preparation of such antigens and the generation of diagnostically useful antibodies that may be used as the first antibody to detect them has been described in detail in WO2010082034, Example 1. Briefly, mice were immunized with lyophilized mycelium of a pathogenic *Aspergillus* strain suspended in PBS, followed by standard production of hybridoma and determination of antibody specificity by ELISA using supernatant of a range of hybridoma.

A serum sample is used for the assay. The patient may be a patient suspected of suffering from IA. Preferably, the patient is a non-haematological patient or a patient treated with antifungal drugs such as posaconazole. Non-haematological patients may include patients having undergone transplantation of solid organs, for example lung, liver or kidney, patients suffering from polytrauma, patients under intensive care and patients suffering from non-haematological tumors.

At least one first antibody binding to said antigen is used according to the present invention. The JF5 antibody disclosed in WO2010082034 may be used or, based on its sequences disclosed in WO2010082034 and in Davies *et al.* (2017), the person skilled in the art can readily design and purify variants of said antibody that also bind to beta-1,5-galactofuranose from mycelium of a pathogenic *Aspergillus* strain, preferably *A. fumigatus.* In a preferred embodiment, the antibody and variants thereof binds specifically to beta-1,5-galactofuranose from *Aspergillus,* preferably *A. fumigatus.* The antibody hjF5 disclosed in Davies *et al.* and variants thereof may also be used. The binding specificity of such an antibody obtained may be confirmed by repeating the experiments in WO2010082034, more specifically Example 3. Briefly, Hartley guinea pigs may be infected by way of exposure to conidia made from a pathogenic *Aspergillus* strain, followed by an immunoassay using the antibody to be characterized.

A second antibody binding to said antigen may be used in alternative assay formats involving the use of the first antibody for immobilizing the antigen and the second antibody for detecting the immobilized antigen. Preferably the antigen is an antigen carrying more than one molecule of beta-1,5-galactofuranose such that both a first antibody and a second antibody binding to beta-1,5-galactofuranose may bind to the antigen at the same time.

In a preferred embodiment, the first and/or second antibody is an antibody binding specifically to the antigen or epitope recognized. In a preferred embodiment, the term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7.

In a preferred embodiment, the diagnostic device or carrier is a bead. In a preferred embodiment, the term "bead" as used herein, refers to a solid bead, preferably made from a carbohydrate or derivate thereof such as agarose or sepharose, to which an antigen or antibody is attached, preferably in a covalent manner. If a bead is used as diagnostic device, it is preferred that the antigen or second antibody has a chemiluminescent label and is detected by chemiluminescence. In a preferred embodiment, the bead is a magnetic bead. Again, the antigen is preferably an antigen carrying more than one molecule of beta-1,5-galactofuranose such that both a first antibody and a second antibody binding to beta-1,5-galactofuranose may bind to the antigen at the same time.

A first antibody binding specifically to beta-1,5-galactofuranose from mycelium of a pathogenic *Aspergillus* strain and optionally, for example in a ELISA immunoassay based on the use of a first antibody for immobilizing the antigen, a second antibody binding specifically to beta-1,5-galactofuranose from mycelium of a pathogenic *Aspergillus* strain may be used according to the present invention such as the antibody JF5 or the antibody AB633-HC3-LC2-T1-1B3-1G10 (hjF5) published by Davies *et al.* (2017) or variants thereof.

In a preferred embodiment, the term "antibody" as used herein, is to be interpreted broadly and comprises not only full-length antibodies but also derivatives and fragments thereof such as Fab, F(ab')2, Fr, ScTv, Fdb and dAb.

In another preferred embodiment, the term "variant" of an antibody, as used herein, refers to a polypeptide or a fragment of said antibody comprising amino acid sequences, preferably a fragment comprising at least 80, more preferably 100, more preferably 110 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antigen of interest, or the fold or structure of the polypeptide are deleted or substituted and/or one or more such essential amino acids are replaced in a conservative manner and/or amino acids are added or deleted such that the biological activity of the polypeptide is at least partially preserved.

The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

In a preferred embodiment, variants may, in addition, comprise chemical modifications, for example labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof.

The variant of the antibody has biological activity. In a preferred embodiment such biological activity is the ability to bind specifically to beta-1,5-galactofuranose, preferably beta-1,5-galactofuranose from mycelium of a pathogenic *Aspergillus* strain, most preferably from *A*. *fumigatus,* which is preferably an antigen secreted exclusively during active growth. Whether or not a variant of the antibody has biological activity may be readily determined in an ELISA as described in Example 1 of the present invention.

In a preferred embodiment, the immunoassay is an ELISA carried out using a microplate. In a preferred embodiment, the microplate is contacted with sample comprising the antigen to be detected, followed by detection of the antigen using a first antibody. In another preferred embodiment, the microplate is coated with a first antibody, followed by incubation with sample comprising antigen and detection of any antigen bound to the first antibody using a second antibody. The second antibody may carry a detectable label or may be detected using a conjugated antibody recognizing the second antibody, and the conjugated antibody carries a detectable label or detected indirectly by binding of a label. In a preferred embodiment, the detectable label is selected from the group comprising a fluorescent, chemiluminescent, radioactive or enzymatically active label.

In a preferred embodiment, the same antibody is used as the first and the second antibody.

In another preferred embodiment, the microplate is coated with an antibody, then contacted with the sample comprising the antigen to be detected, followed by incubation in the presence of antigen comprising a detectable label which then competes with the antigen to be detected.

Disclosed herein is a kit, preferably for diagnosing aspergillosis. Such a kit is a container that comprises specific reagents required to practice the inventive method, in particular a carrier selected from the group consisting of an ELISA microplate and a bead, coated with antigen, preferably in the form of antigen immobilized via a first antibody binding specifically to the antigen, or a first antibody binding specifically to a proteinaceous antigen derived from mycelium of a pathogenic *Aspergillus* strain. Any antigen coated may be antigen carrying a detectable label. Optionally the kit comprises to one or more solutions required to practice the inventive method in addition. The solution is or the solutions are preferably selected from or are all solutions from the group comprising sample dilution buffer, washing buffer and buffer comprising a means for detecting a bound antibody, such as a secondary antibody and optionally a means for detecting the latter. The kit may comprise a second antibody binding specifically to the antigen, which second antibody is in a solution or in a solid form made to prepare such a solution. In a preferred embodiment, the first and the second antibody is the same antibody, and it is in a more preferred embodiment the antibody binding to the JF5 antigen. The kit may comprise at least three, four or five different solutions having different, known concentrations of beta-1,5-galactofuranose or an *Aspergillus* cell or a derivative thereof for setting up a calibration curve or as a positive or negative control.

Furthermore, the kit may comprise instructions detailing how to use the kit and the inventive diagnostically useful carrier.

In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from IA in the past, at the time of the diagnosis or in the future, to find out how the disease is progressing or is likely to progress in the future or to evaluate the responsiveness of a patient with regard to a certain treatment, for example the administration of suitable drugs such as antifungal drugs. In other words, the term "diagnosis" comprises not only diagnosing, but also prognosticating and/or monitoring the course of a disease or disorder.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. The term "diagnosis" may also refer to a method or agent used to choose the most promising treatment regimen for a patient. In other words, the method or agent may relate to selecting a treatment regimen for a subject.

In a preferred embodiment, the term "diagnostic reliability", as used herein refers to the combination of sensitivity and specificity of an immunoassay.

In a preferred embodiment, the term "sensitivity", as used herein, refers to the ratio of positive samples correctly recognized as such by carrying out the assay of interest and the total number of samples examined. In other words, an assay has 100% sensitivity if all samples from infected patients give a positive result, *i.e.* there are no false negative results.

In a preferred embodiment, the term "specificity", as used herein, refers to the ratio of negative samples correctly recognized as such by carrying out the assay of interest and the total number of samples examined. In other words, an assay has 100% specificity if all samples from healthy subjects give a negative result, i.e. there are no false positive results.

According to the present invention, the sample is subjected to a denaturing step, preferably a boiling step prior to the step contacting the sample with the antibody. A chelating reagent such as EDTA may be present. This may be accomplished by elevating the temperature in the sample such that the liquid sample solution starts boiling, for example by placing the sample in a boiling water bath. In a more preferred embodiment, the sample is kept for at least 1, 2, 3, 4, 5 or 10 minutes, preferably 3 minutes at a temperature that will cause the sample to boil, for example 100 °C. Subsequently, any insoluble components may be separated from the liquid sample, preferably by subjecting the boiled sample to centrifugation. The supernatant is then used for the next steps as the sample for the immunoassay. Alternatively, the denaturing step may be accomplished by adding a denaturing agent, for example 6 molar guanidinium hydrochloride, which must, however, be removed or its concentration must be reduced such that the binding activity antibodies used in the method according the present invention is maintained.

### Example 1:

### 1. Patients:

A cohort comprising 38 Patients suffering from *confirmed* IA was recruited. Patients were diagnosed by expert clinicians based on the EORTC/MSG guidelines. They included a wide spectrum of subjects comprising both haematooncologic patients and patients under intensive care. A range of non-infectious patients were used as controls.

From each patient, several samples taken at various time points were measured to exclude the possibility that antibody titers were too low for detection as a result of the disease being at an early stage.

Blood samples from a control cohort of 79 healthy subjects suspected of suffering from a tick-borne disease were tested as a negative control.

### 2. Preparation of sample

300 µl of a sample or calibrator (such as human serum spiked with varying amounts of *A. fumigatus* antigen as in Example 3) were mixed using 100 µl sample diluent (PBS comprising 0.1 M EDTA). This mixture was then heated for 3 minutes in a boiling water bath, spun down (10 minutes at 10.000 x g), followed by recovering the supernatant, which was subsequently used.

### 3. Preparation of antibody-coated wells

JF5 antibody described in WO2010/082034 was immobilized on the surface of wells from a microplate (NUNC MaxiSorp) using standard methods.

### 4. Assay

100 µl of said mixture comprising calibrators, controls or sample supernatant were transferred in a microplate well coated with JF5 antibodies

For the incubation step, wells were covered and kept for one hour at room temperature (18°C to 25°C) at medium speed (approximately 450 rpm) on an orbital shaker. Samples were removed, followed by addition of 300 µl per well of washing buffer (available as ZE1120 from EUROIMMUN AG, Lübeck) and an incubation for 30 to 60 seconds per washing cycle. Three washing cycles were carried out.

Washing buffer was removed, followed by addition of 100 µl per well of biotinylated JF5 antibody in EUROIMMUN Probenpuffer (Catalogue number ZE1100). Wells were covered and incubated at room temperature for one hour on an orbital shaker (450rpm). The washing steps were repeated.

In a third incubation step 100 µl Enzyme conjugate (EUROIMMUN AG, ZE 6911-2) is added and incubated 30 min at room temperature on an orbital shaker (450 rpm).

Washing steps were repeated, followed by addition of 100 µl per well of chromogen and substrate (Standard ELISA TMB substrate, available from EUROIMMUN AG, catalogue number ZE 1200) and incubation for 15 minutes at room temperature. The reaction was stopped by addition of 100 µl per well of stop solution (EUROIMMUN AG, catalogue number Cat# ZE 1210).

Absorbance of the solution was measured at 450 nm.

Samples were determined as positive if the absorbance of 450 nm was beyond a cutoff threshold calculated by routine methods.

### 5. Galactomannan assay obtained from Bio-Rad:

The Platelia Aspergillus AG assay from Bio-Rad (Catalogue No. 62794) was carried out in parallel according to manufacturer's instructions.

### 6. Results

Using the conventional galactomannan assay (Bio-Rad), 20 out of 38 patients were identified as positive (sensitivity: 53 %). Eight patients were misdiagnosed.
Using the assay according to the present invention, 25 out of 38 patients were diagnosed correctly (sensitivity: 66 %). Only two patients were misdiagnosed. 78 out of 79 healthy subjects were tested negative (specificity: 98.7 %).

### Example 2:

In separate study, it was investigated at which time point following infection the two assays yield correct positive results.

Several samples per week were collected from patients suffering from leukemia and a probable aspergillosis and analyzed as in Example 1. All these patients were eventually tested positive using assays, the conventional assay and the assay according to the present invention.

Using the assay according to the present invention, 3 patients could be diagnosed earlier using the assay according to the present invention. The delay until the conventional galactomannan assay yielded positive results was four to 29 days.

Only in one case yielded the conventional assay a positive result before the assay according to the present invention did.

### Conclusions:

The assay according to the present invention, when compared to the conventional assay using sera from patient suffering from a variety of diseases, is superior in terms of sensitivity and time until a correct positive result may be obtained.

### Example 3:

A reference panel made from human plasma spiked with increasing concentrations of *Aspergillus fumigatus* extract, five negative samples from healthy blood donors and five samples tested using the BioRad Galactomannan Assay were subjected to the assay described in Example 1.

The results are shown in Table 1.

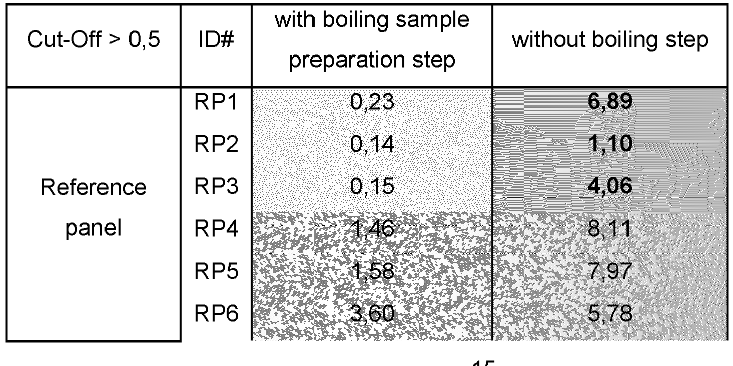

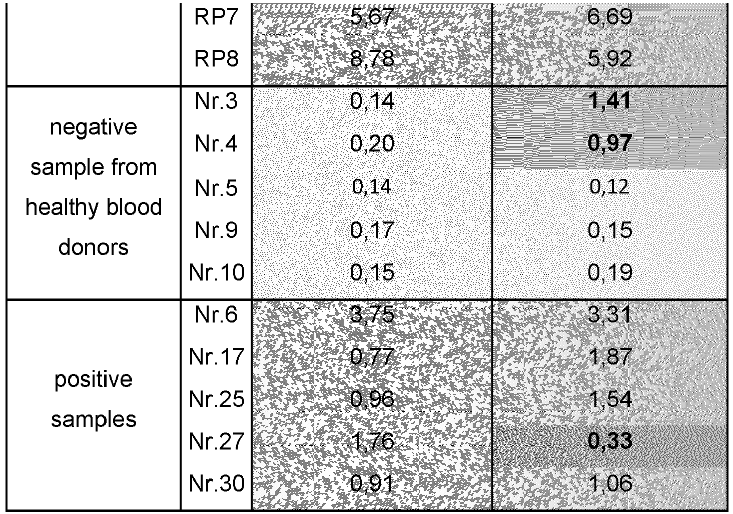

The cut off value was 0.5, i.e. signals < 0.5 indicate a negative result, signals > 0.5 a positive result. Wrong results obtained without boiling step are in bold.

The reference panel comprises processed human plasma without antigen (RP1), CPD-Plasma of two different human blood donors without antigen (RP2, RP3), processed human plasma with 0,08 ng/ml *Aspergillus f.* lysate (RP4), processed human plasma with 0,12 ng/ml *Aspergillus f.* lysate (RP5), processed human plasma with 0,18 ng/ml *Aspergillus f.* lysate (RP6), processed human plasma with 0,5 ng/ml *Aspergillus f.* lysate (RP7), processed human plasma with 0,6 ng/ml *Aspergillus f.* lysate (RP8).

Basically the assay according to the present invention shows increasing signals in line with the increasing concentration of antigen in the samples. By contrast, results are scattered and appear to be arbitrary if the boiling step is omitted at low concentrations, showing that the boiling step helps to avoid false positive results at lower concentration of the antigen, i.e. increases the specificity of the test.

Two of the negative samples are false positive if processed without the boiling step, but are correctly evaluated as negative if the assay according to the present invention including the boiling step is used. This corroborates the finding that the specificity is increased.

One out of five samples appears to be false negative if the boiling step is omitted, but not if the boiling step is included.

Overall, these data show that denaturing the sample increases the diagnostic reliability, in particular the specificity, and reproducibility of the assay.

### Example 4:

In separate study, it was investigated at which time point following infection the two assays yield correct positive results, again, using a larger number of patients. Again, the JF5 assay was used based on the protocol described in Example 1, and the GM assay was used according to the manufacturer's instructions.

A cohort of 31 patients diagnosed as positive or probably positive according to the medical guidelines were subjected to testing on multiple days. In addition to a sample on day 0 (time of the diagnosis), additional samples prior to or after day 0 were analyzed.

Among 11 out of 31 patients, the assay based on JF5 yielded (correct) positive results over a longer time period. For eight patients, a positive diagnosis could be accomplished using the JF5-based assay at an earlier time compared to the GM assay. For four patients, the test result remained positive for a longer period of time after day 0.

By contrast, in the case of only three patients out of 31 a correct positive result could be obtained in a longer time period using the GM test compared to the test based on JF5. Two of these patients could be diagnosed as positive for a longer time after day 0, whereas one patient could be diagnosed as positive earlier before day 0 compared to the result of the JF5-based assay.

In summary, these results show that a longer time period is available for diagnosing patients correctly as positive if the JF5-based assay is used rather than the GM-based assay. Using JF5, both the time until a positive result can be obtained is shorter and the period until a positive result can no longer be obtained is extended.

## Claims

1. A method for diagnosing an *Aspergillus* infection in a patient, comprising the step detecting in a sample beta-1,5-galactofuranose from mycelium of a pathogenic *Aspergillus* strain,
wherein the sample is contacted with a first antibody binding specifically to said antigen and
wherein any antigen bound to said first antibody is detected by way of an immunoassay selected from the group consisting of an ELISA, wherein the first antibody is coated on a microplate, and a bead-based assay, wherein the first antibody is coated on a bead
wherein the immunoassay is not a membrane-based immunoassay selected from the group consisting of lateral flow device, western blot, dot blot and line blot,
wherein the sample is a serum sample,
**characterized in that** the sample is subjected to a denaturing step prior to contacting the sample with the antibody.

2. A use of an antibody binding specifically to beta-1,5-galactofuranose from mycelium of a pathogenic *Aspergillus* strain for increasing the diagnostic reliability of an immunoassay for the diagnosis of an *Aspergillus* infection in a patient comprising the step detecting said antigen in a sample,
wherein the sample is contacted with a first antibody to said antigen followed by detection of any antigen bound to said first antibody by way of an immunoassay selected from the group consisting of an ELISA, wherein the first antibody is coated on a microplate, and a bead-based assay, wherein the first antibody is coated on a bead,
wherein the immunoassay is not a membrane-based immunoassay selected from the group consisting of lateral flow device, western blot, dot blot and line blot,
wherein the sample is a serum sample,
**characterized in that** the sample is subjected to a denaturing step prior to contacting the sample with the antibody.

3. The method or use according to any of claims 1 to 2, wherein the immunoassay is a sandwich ELISA.

4. The method or use according to any of claims 1 to 3, wherein the first antibody is a monoclonal antibody.

5. The method or use according to any of claims 1 to 4, wherein the first antibody is the antibody JF5.

6. The method or use according to any of claims 1 to 5, wherein the *Aspergillus* strain is selected from the group *A. fumigatus, A. flavus, A. niger, A. terreus* and *A. nidulans* and is preferably *A. fumigatus* AF293.

7. The method or use according to any of claims 1 to 6, wherein the method is for diagnosing an invasive aspergillosis.

8. The method or use according to any of claims 1 to 7, wherein the denaturing step is a boiling step.

9. The method or use according to any of claims 1 to 8, wherein the antigen is detected using a second antibody, which is preferably the same antibody as the first antibody.

10. The use according to any of claims 2 to 9 wherein the use is for increasing the specificity, preferably in the diagnosis of a human patient treated with an anti-fungal drug, more preferably Posaconazole.

11. The use according to any of claims 2 to 10, wherein the use is for increasing the diagnostic reliability at an early stage of the infection.

12. The method or use according to claim 9, wherein the second antibody carries a detectable label.

13. The method or use according to claim 9 wherein the second antibody is detected using a conjugated antibody recognizing the second antibody and wherein the conjugated antibody carries a detectable label.

14. The method or use according to any of claims 12 or 13 wherein the label is selected from the group consisting of a fluorescent, chemiluminescent, radioactive or enzymatically active label.

## Patentansprüche

1. Verfahren zum Diagnostizieren einer *Aspergillus-*Infektion bei einem Patienten umfassend den Schritt Nachweisen von beta-1,5-Galactofuranose aus Myzel eines pathogenen *Aspergillus-*Stamms in einer Probe,
wobei die Probe mit einem ersten Antikörper kontaktiert wird, der spezifisch an dieses Antigen bindet, und
wobei jegliches Antigen, das an den ersten Antikörper gebunden ist, durch einen Immunassay nachgewiesen wird, der aus der Gruppe ausgewählt ist, die aus einem ELISA, wobei der erste Antikörper auf einer Mikroplatte als Beschichtung aufgetragen ist, und einem beadbasierten Assay, wobei der erste Antikörper auf einen Bead als Beschichtung aufgetragen ist, besteht,
wobei es sich bei dem Immunassay nicht um einen membranbasierten Immunassay, der aus der Gruppe ausgewählt ist, die aus Lateral Flow-Assay, Western Blot, Dot Blot und Line Blot besteht, handelt,
wobei die Probe eine Serumprobe ist,
**dadurch gekennzeichnet, dass** die Probe einem Denaturierungsschritt unterzogen wird, bevor die Probe mit dem Antikörper kontaktiert wird.

2. Verwendung eines Antikörpers, der spezifisch an beta-1,5-Galactofuranose aus Myzel eines pathogenen *Aspergillus-*Stamms bindet zum Erhöhen der diagnostischen Zuverlässigkeit eines Immunassays für die Diagnose einer *Aspergillus-*Infektion bei einem Patienten
umfassend den Schritt Nachweisen dieses Antigens in einer Probe,
wobei die Probe mit einem ersten Antikörper gegen dieses Antigen kontaktiert wird, gefolgt vom Nachweis jeglichen Antigens, das an diesen ersten Antikörper gebunden ist, durch einen Immunassay, der aus der Gruppe ausgewählt ist, die aus einem ELISA, wobei der erste Antikörper auf eine Mikroplatte als Beschichtung aufgetragen ist, und einem beadbasierten Assay, wobei der erste Antikörper auf einen Bead als Beschichtung aufgetragen ist, besteht,
wobei es sich bei dem Immunassay nicht um einen membranbasierten Immunassay, der aus der Gruppe ausgewählt ist, die aus Lateral Flow-Assay, Western Blot, Dot Blot und Line Blot besteht, handelt,
wobei die Probe eine Serumprobe ist,
**dadurch gekennzeichnet, dass** die Probe einem Denaturierungsschritt unterzogen wird, bevor die Probe mit dem Antikörper kontaktiert wird.

3. Das Verfahren oder die Verwendung nach einem der Ansprüche 1 bis 2, wobei der Immunassay ein Sandwich-ELISA ist.

4. Das Verfahren oder die Verwendung nach einem der Ansprüche 1 bis 3, wobei der erste Antikörper ein monoklonaler Antikörper ist.

5. Das Verfahren oder die Verwendung nach einem der Ansprüche 1 bis 4, wobei der erste Antikörper der Antikörper JF5 ist.

6. Das Verfahren oder die Verwendung nach einem der Ansprüche 1 bis 5, wobei der *Aspergillus-*Stamm aus der Gruppe *A. fumigatus, A. flavus, A. niger, A. terreus* und *A*. *nidulans* ausgewählt ist und bevorzugt *A. fumigatus* AF293 ist.

7. Das Verfahren oder die Verwendung nach einem der Ansprüche 1 bis 6, wobei es sich bei dem Verfahren um ein Verfahren zur Diagnose einer invasiven Aspergillose handelt.

8. Das Verfahren oder die Verwendung nach einem der Ansprüche 1 bis 7, wobei der Denaturierungsschritt ein Kochschritt ist.

9. Das Verfahren oder die Verwendung nach einem der Ansprüche 1 bis 8, wobei das Antigen unter Verwendung eines zweiten Antikörpers nachgewiesen wird, der bevorzugt der gleiche Antikörper wie der erste Antikörper ist.

10. Die Verwendung nach einem der Ansprüche 2 bis 9, wobei es sich bei der Verwendung um eine Verwendung zur Erhöhung der Spezifität handelt, bevorzugt bei der Diagnose eines menschlichen Patienten, der mit einem Antimykotikum behandelt wird, bevorzugter Posaconazol.

11. Die Verwendung nach einem der Ansprüche 2 bis 10, wobei es sich bei der Verwendung um eine Verwendung zur Erhöhung der diagnostischen Zuverlässigkeit in einem frühen Stadium der Infektion handelt.

12. Das Verfahren oder die Verwendung nach Anspruch 9, wobei der zweite Antikörper eine nachweisbare Markierung trägt.

13. Das Verfahren oder die Verwendung nach Anspruch 9, wobei der zweite Antikörper unter Verwendung eines konjugierten Antikörpers nachgewiesen wird, der den zweiten Antikörper erkennt, und wobei der konjugierte Antikörper eine nachweisbare Markierung trägt.

14. Das Verfahren oder die Verwendung nach einem der Ansprüche 12 oder 13, wobei die Markierung aus der Gruppe ausgewählt ist, die aus einer fluoreszenzfähigen, chemilumineszenzfähigen, radioaktiven oder enzymatisch aktiven Markierung besteht.

## Revendications

1. Procédé de diagnostic d'une infection par *Aspergillus* chez un patient, comprenant l'étape de détection dans un échantillon de bêta-1,5-galactofuranose de mycélium d'une souche pathogène d*'Aspergillus,*
dans lequel l'échantillon est mis en contact avec un premier anticorps se liant spécifiquement audit antigène et
dans lequel l'antigène lié audit premier anticorps est détecté au moyen d'un immunoessai choisi dans le groupe constitué d'un ELISA, dans lequel le premier anticorps est revêtu sur une microplaque, et d'un essai à base de billes, dans lequel le premier anticorps est revêtu sur une bille,
dans lequel l'immunoessai n'est pas un immunoessai à base de membrane choisi dans le groupe constitué d'un dispositif à écoulement latéral, d'un transfert western, d'un transfert en point et d'un transfert en ligne,
dans lequel l'échantillon est un échantillon de sérum,
**caractérisé en ce que** l'échantillon est soumis à une étape de dénaturation avant la mise en contact de l'échantillon avec l'anticorps.

2. Utilisation d'un anticorps se liant spécifiquement au bêta-1,5-galactofuranose de mycélium d'une souche pathogène d*'Aspergillus* pour augmenter la fiabilité de diagnostic d'un immunoessai pour le diagnostic d'une infection par *Aspergillus* chez un patient
comprenant l'étape de détection dudit antigène dans un échantillon,
dans laquelle l'échantillon est mis en contact avec un premier anticorps contre ledit antigène suivi par la détection de l'antigène lié audit premier anticorps au moyen d'un immunoessai choisi dans le groupe constitué d'un ELISA, dans lequel le premier anticorps est revêtu sur une microplaque, et d'un essai à base de billes, dans lequel le premier anticorps est revêtu sur une bille,
dans lequel l'immunoessai n'est pas un immunoessai à base de membrane choisi dans le groupe constitué d'un dispositif à écoulement latéral, d'un transfert western, d'un transfert en point et d'un transfert en ligne,
dans lequel l'échantillon est un échantillon de sérum,
**caractérisé en ce que** l'échantillon est soumis à une étape de dénaturation avant la mise en contact de l'échantillon avec l'anticorps.

3. Procédé ou utilisation selon l'une quelconque des revendications 1 à 2, dans lequel l'immunoessai est un ELISA en sandwich.

4. Procédé ou utilisation selon l'une quelconque des revendications 1 à 3, dans lequel le premier anticorps est un anticorps monoclonal.

5. Procédé ou utilisation selon l'une quelconque des revendications 1 à 4, dans lequel le premier anticorps est l'anticorps JF5.

6. Procédé ou utilisation selon l'une quelconque des revendications 1 à 5, dans lequel la souche d'*Aspergillus* est choisie dans le groupe de *A. fumigatus*, *A. flavus, A. niger, A. terreus* et *A. nidulans* et est de préférence *A. fumigatus* AF293.

7. Procédé ou utilisation selon l'une quelconque des revendications 1 à 6, le procédé étant pour diagnostiquer une aspergillose invasive.

8. Procédé ou utilisation selon l'une quelconque des revendications 1 à 7, dans lequel l'étape de dénaturation est une étape d'ébullition.

9. Procédé ou utilisation selon l'une quelconque des revendications 1 à 8, dans lequel l'antigène est détecté au moyen d'un deuxième anticorps, qui est de préférence le même anticorps que le premier anticorps.

10. Utilisation selon l'une quelconque des revendications 2 à 9, l'utilisation étant pour augmenter la spécificité, de préférence dans le diagnostic d'un patient humain traité avec un médicament antifongique, plus préférablement le posaconazole.

11. Utilisation selon l'une quelconque des revendications 2 à 10, l'utilisation étant pour augmenter la fiabilité de diagnostic à un stade précoce de l'infection.

12. Procédé ou utilisation selon la revendication 9, dans lequel le deuxième anticorps porte un marqueur détectable.

13. Procédé ou utilisation selon la revendication 9, dans lequel le deuxième anticorps est détecté au moyen d'un anticorps conjugué reconnaissant le deuxième anticorps et dans lequel l'anticorps conjugué porte un marqueur détectable.

14. Procédé ou utilisation selon l'une quelconque des revendications 12 ou 13, dans lequel le marqueur est choisi dans le groupe constitué d'un marqueur fluorescent, chimioluminescent, radioactif ou enzymatiquement actif.
